Europäisches Patentamt

European Patent Office

Office européen des brevets

(19)

(11) Publication number: **0 389 377**
**A1**

## (12) EUROPEAN PATENT APPLICATION

(21) Application number: **90400796.0**

(22) Date of filing: **22.03.90**

(51) Int. Cl.5: **C12P 21/02, C12N 1/38, C12N 1/06**

(30) Priority: **22.03.89 US 327123**

(43) Date of publication of application:
**26.09.90 Bulletin 90/39**

(84) Designated Contracting States:
**AT BE CH DE DK ES FR GB GR IT LI LU NL SE**

(71) Applicant: **HER MAJESTY IN RIGHT OF CANADA AS REPRESENTED BY THE NATIONAL RESEARCH COUNCIL OF CANADA**
**Montreal Road**
**Ottawa Ontario K1A 0R6(CA)**

(72) Inventor: **Shen, Shi-Hsiang**
**161 Chartwell Circle**
**Beaconsfield, Quebec H9W 1C2(CA)**
Inventor: **Slilaty, Steve N.**
**3550 Jeanne-Mance Nr. 2412**
**Montreal, Quebec H2X 3P7(CA)**
Inventor: **Bastien, Lison**
**625 Ste-Marguerite**
**Montreal, Quebec H4C 2X3(CA)**

(74) Representative: **Peaucelle, Chantal et al**
**S.C. Ernest Gutmann - Yves Plasseraud 67, boulevard Haussmann**
**F-75008 Paris(FR)**

(54) Excretion of proteins from yeast cells.

(57) A process for the production of engineered biological products by yeast cells which comprises continuously growing the yeast cells in a growth medium in the presence of an enzyme capable of permeabilizing the yeast cell wall to allow release of the biological products from the inside of the cell wall into the growth medium. The desired products are then recovered from the growth medium.

EP 0 389 377 A1

Excretion of proteins from yeast cells.

## BACKGROUND OF THE INVENTION

Different microorganisms, among which are bacteria and yeasts, can be used as host organisms for different plasmids containing a nucleotide sequence coding for a specific protein. Among these microorganisms, yeasts are presently preferred and currently used. For example, European patent application publication No. 0 106 828 discloses the use of Saccharomyces cerevisiae strains for the production of hepatitis B virus surface antigen (HBsAg) and European patent application publication No. 0 103 409 relates to the production of alpha-1-antitrypsin from yeast plasmids.

Protein production in yeast strains indeed presents substantial advantages over production in bacterial strains. These advantages result from the rather easy growth of yeast in large scale fermenters and from the fact that, contrary to bacteria, yeasts do appear to resemble mammalian cells in their capacity to appropriately fold expressed proteins and add carbohydrate groups to newly synthesized proteins.

Nevertheless the extraction and purification of the protein from yeast cells do present technical problems due to the rather complex chemical composition of the yeast cell and more particularly to the presence of high lipid levels when the yeast growth is extended in order to improve the yield of polypeptide production.

For example, the Saccharomyces cell wall is thought to consist of 3 layers, (a) an inner layer of alkali-insoluble $\beta$-glucan, (b) a middle layer of alkali-soluble $\beta$-glucan and (c) an outer layer of glycoprotein in which the carbohydrate consists of phosphorylated mannan; beneath the cell wall is a cytoplasmic membrane consisting of a very complex mixture of neutral lipids (mono-, di-and tri-glycerides), free and esterified sterols, complex sphingolipids, glycerophosphatides and neutral as well as acidic glycolipids; the nucleus contains DNA, various species of RNA and a polyphosphate; vacuoles may contain a great variety of components of both high and low molecular weights, they serve as storage vesicles for a number of hydrolytic enzymes; the mitochondria are rich in lipid, phospholipid and ergosterol components of the membrane system and the cytoplasm contains large quantities of ribosomes, polyphosphates, glycogen and a number of glycolytic enzymes. Most yeast cells (such as Saccharomyces species) also contain some lipids in the form of globules, and amount of which does increase in extended cultures.

A number of multi-steps processes have been disclosed for the extraction and purification of proteins from different sources. Examples referring to proteins produced by engineered microorganisms are those published by T. Staehelin et al. (J. Biol. Chem., 256:9750-54, 1981), K. Murray et al. (Embo J., 3:645-650, 1984) and R.A. Hitzeman et al. (Nucl. Acids Res., 11:2745-2763, 1983).

Typically, when the produced protein is inside the cell, those different processes involve 3 series of steps.

In the first series of steps, the desired protein is removed from the cell interior. Therefore, the cells are either lysed (e.g. by enzymatic treatment) or disrupted (e.g. by mechanical forces (such as shearing with e.g. X-press or French press, or by shaking with glass beads)), and eventually by addition of a detergent (see, for instance, K. Murray et al. loc. cit. and R.A. Hitzemann et al. loc. cit.).

In the second series of steps, the solution from lysed or destructed cells is enriched in the desired protein by fractional precipitation e.g. by addition of ammonium sulfate and/or polyethylene glycol.

Finally, in the third series of steps, substantially all contaminants are eliminated; e.g. by one or several operations selected from the group comprising ultrafiltration, ion exchange chromatography, gel filtration chromatography, isopycnic gradient centrifugation, and other purification methods.

In such processes, it is obvious that contaminants such as accompanying proteins (cited by R.A. Hitzemann et al. loc. cit.), nucleic acids and lipids and more particularly high lipid levels have a harmful influence on at least one of the steps of the third series (e.g. ultrafiltration and column chromatography) and the proteolytic enzymes must be eliminated rapidly. Moreover, it has also been noticed that ammonium sulfate precipitation is not possible without a previous rough delipidation because lipids interfere with this precipitation.

Therefore, it is of prime importance to dispose of a method wherein most of the contaminants are eliminated and wherein substantially pure desirable products such as HBsAg may be efficiently produced and recovered.

During a hepatitis B virus infection, in addition to the infectious virion called Dane particle, hepatitis B surface antigen (HBsAg), the major coat protein of hepatitis B virus, is massively secreted into the serum of patients as a 22 nm subviral particle (Robinson, W.S., and L.I. Lutwick, The virus of hepatitis type B, N. Engl. J. Med., 295:1168-1175, (1976)). These particles consist of a membrane structure into which a major viral protein, S, and two related minor proteins, preS1 and preS2, are embedded (K.H. Heermann, U. Goldmann, W. Schwartz, T. Seyffarth, H. Baumgarten, and W.H. Gerlich, Large surface proteins of hepatitis B virus containing the preS sequence, J. Virol., 52:396-402, 1984). The S polypeptide itself is hydrophobic (Peterson, D., Isolation and characterization of the major protein and glycoprotein of hepatitis B surface antigen, J. Biol. Chem., 256:6975-6983, 1981) and contains internal uncleaved signal sequences which direct portions of the molecule across the membrane (Eble, B.E., D.D. MacRae, V.R. Lingappa, and D. Ganem, Multiple topogenic sequences determine the transmembrane orientation of hepatitis B surface antigen, Mol. Cell. Biol., 7:3591-3601, (1987); Eble, B.E., V.R. Lingappa, and D. Ganem, Hepatitis B surface antigen: an unusual secreted protein initially synthesized as a transmembrane polypeptide, Mol. Cell. Biol., 6: 1454-1463, (1986)). The initial product of HBsAg synthesis is an integral transmembrane protein (Eble, B.E., V.R. Lingappa, and D. Ganem, Hepatitis B surface antigen: an unusual secreted protein initially synthesized as a transmembrane polypeptide, Mol. Cell. Biol., 6:1454-1463, (1986)).

In animal cells, it has been shown that the recombinant HBsAg is assembled into a 22 nm lipoprotein particle and secreted into the medium (Crowley, C.W., C.C. Liu, and A.D. Levinson, Plasmid-directed synthesis of hepatitis B surface antigen in monkey cells, Mol. Cell. Biol., 3:44-55, (1983); Dubois, M.F., C. Pourcel, S. Rousset, C. Chany, and P. Tiollais, Excretion of hepatitis B surface antigen particles from mouse cells transformed with cloned viral DNA, Proc. Natl. Acad. Sci USA, 77:4549-4553, (1983); Moriarty, A.M., B.H. Hoyer, J.W. Shih, J.L. Gerin, and D.H. Hamer, Expression of the hepatitis B virus surface antigen gene in cell culture by using a simian virus 40 vector, Proc. Natl. Acad. Sci. USA, 78: 2606-2610, (1981); Persing, D.H., H.E. Varmus, and D. Ganem, Inhibition of secretion of hepatitis B surface antigen by a related presurface polypeptide, Science, 234: 1388-1391, (1986)).

HBsAg particles are generated by aggregation of antigen monomers, associated with cellular lipids, in the endoplasmic reticulum (ER), followed by budding into the lumen of the ER, and ultimately passing through the plasma membrane into the medium (Crowley, C.W., C.C. Liu, and A.D. Levinson, Plasmid-directed synthesis of hepatitis B surface antigen in monkey cells, Mol. Cell. Biol., 3:44-55, (1983); Dubois, M.F., C. Pourcel, S. Rousset, C. Chany, and P. Tiollais, Excretion of hepatitis B surface antigen particles from mouse cells transformed with cloned viral DNA, Proc. Natl. Acad. Sci USA, 77:4549-4553, (1980); Eble, B.E., D. MacRae, V.R. Lingappa, and D. Ganem, Multiple topogenic sequences determine the transmembrane oritentation of hepatitis B surface antigen, Mol. Cell. Biol. 7:3591-3601, (1987), Eble, B.E., V.R. Lingappa, and D. Ganem, Hepatitis B surface antigen: an unusual secreted protein initially synthesized as a transmembrane polypeptide, Mol. Cell. Biol., 6: 1454-1463, (1986); Moriarty, A.M., B.H. Hoyer, J.W. Shih, J.L. Gerin, and D.H. Hamer, Expression of the hepatitis B virus surface antigen gene in cell culture by using a simian virus 40 vector, Proc. Natl. Acad. Sci. USA, 78:2606-2610, (1981); Patzer, E.J., G.R. Nakamura, C.C. Simonsen, A.D. Levinson, and R. Brands, Intracellular assembly and packaging of hepatitis B surface antigen particles occur in the endoplasmic reticulum, J. Virol. 58:884-892, (1986); Patzer, E.J., G.R. Nakamura, and A. Yaffe, Intracellular transport and secretion of hepatitis B surface antigen in mammalian cells, J. Virol., 51:346-353 (1984)). In yeast, however, the recombinant HBsAg proteins, though capable of assembling into 22 nm lipoprotein particles, have only been found as intracellular products as described by Cregg, J.M., J.F. Tschopp, C. Stillman, R. Siegel, M. Akong, W.S. Craig, R.G. Buckholz, K.R. Madden, P.A. Kellaris, G.R. Davis, B.L. Smiley, J. Cruze, R. Torregrossa, G. Velicelebi, and G.P. Thill, High-level expression and efficient assembly of hepatitis B surface antigen in the methylotrophic yeast, Pichia pastoris, Biotechnology 5:479-485, (1987); Hitzeman R.A., C.Y. Chen, F.E. Hagie, E.J. Patzer, C.C. Liu, D.A. Estell, J.V. Miller, A. Yaffe, D.G. Kleid, A.D. Levinson, and H. Oppermann, Expression of hepatitis B virus surface antigen in yeast, Nucleic Acids Res. 11:2745-2763, (1983).

The recombinant HBsAg protein found as an intracellular product was also described by Kitano, K., M. Nakao, Y. Itho, and Y. Fujisawa, Recombinant hepatitis B virus surface antigen P31 accumulates as particles in Saccharomyces cerevisiae, Biotechnology 5:281-283 (1987); Langley, K.E., K.M. Egan, J.M. Barendt, C.G. Parker, and G.A. Bitter, Characterization of purified hepatitis B surface antigen containing pre-S(2) epitopes expressed in Saccharomyces cerevisiae, Gene 67:229-245, (1988), Miyanohara, A., A. Toh-e, C. Nozaki, F. Hamasa, N. Ohtomo, and K. Matsubara, Expression of hepatitis B surface antigen gene in yeast, Proc. Natl. Acad. Sci. USA 80:1-5, (1983); Valenzuela, P., D. Coit, and C.H. Kuo, Synthesis and assembly in yeast of hepatitis B surface antigen particles containing the polyalbumin receptor, Biotechnology 3:317-320, (1985); Valenzuela, P., A. Medina, W.J. Rutter, G. Ammerer, and B.D. Hall, Synthesis and assembly of hepatitis B virus surface antigen particles in yeast, Nature 298:347-350, (1982). No extracellular HBsAg particles have thus far been isolated. This is a major disadvantage for production of HBsAg vaccine from yeasts particularly in regards to downstream processing (i.e. purification) and immunogenicity of the antigens.

The efficient synthesis and secretion of hepatitis B surface antigen (HBsAg) is of considerable

EP 0 389 377 A1

importance because of the numerous possible uses of HBsAg in the medical field. However, although HBsAg has been successfully expressed in various yeast systems and the first generation of recombinant HBsAg vaccine has been produced from Saccharomyces cerevisiae, all current HBsAg particles can only be isolated from broken cells. This is a major drawback for production of HBsAg because of the difficulty of cell breakage and product purification.

It would be highly desirable to have a method for removal of proteins from their intracellular locations in their host without having to break the cell walls, that is a means to permeabilize the host cell wall in order to excrete the desired proteins into the medium.

## SUMMARY OF THE INVENTION

In accordance with the present invention, there is provided means for favoring excretion of proteins trapped in the periplasm of yeast cells into the culture medium in which the cells are grown.

The present invention thus relates to a process for the excretion and production of biological products from yeast cells. The products comprises continuously growing the yeast cells in a growth medium in the presence of at least one enzyme capable of permeabilizing the cell wall of the yeast cells, thereby allowing release of the biological products from the inside of the cell wall into said growth medium. The desired products are then recovered from the growth medium.

In order to illustrate the applicability of the process of the present invention, the methylotrophic yeast, Hansenula polymorpha, has been developed as a new host for expression of heterologous proteins. A fermentation technique for H. polymorpha has already been developed in an inexpensive medium with high biomass for production of single cell protein, using methanol as a sole carbon source (U.S.P. 4,414,329).

Two kinds of expression vectors have been constructed; one based on autonomously replicating plasmids and the other, an integration vector, based on homologous recombination. A methanol-regulated promoter derived from the methanol oxidase (MOX) gene has been engineered to direct foreign gene expression. Other promoters, for example that of dihydroxyacetone synthase (DHAS), catalase, and formate dehydrogenase (FMD), which are involved in methanol metabolism, can be used for expression of foreign genes as they are synthesized in abundance and regulated by methanol.

In H. polymorpha, the HBsAg synthesized can be exported outside the cell membrane into the periplasm, a characteristic similar to animal systems. In accordance with the present invention, the HBsAg trapped in the periplasm has been excreted into the culture medium by adding a glucanase such as $\beta$-1,3-glucanase to the medium. HBsAg proteins have been purified directly from the medium using a simple process. Its property of 22 nm particle has been verified by electron microscopic observation and its identity and molecular weight have been assessed by immunoblotting.

As mentioned above, one of the advantages of excreting HBsAg into the medium is to facilitate the downstream processing of HBsAg production. The others include the possibility of increasing the immunogenicity of the recombinant products since the intracellular HBsAg isolated from S. cerevisiae is not a fully disulfide-bonded complex particle, different from the plasma-derived antigen in appearance, chemical and immunological properties (D.E. Wampler, E.D. Lehman, J. Boger, W.J. McAleer, and E.M. Scolnick, Multiple chemical forms of hepatitis B surface antigen produced in yeast, Proc. Natl. Acad. Sci. U.S.A., 82:6830-6834, 1985). Moreover, the excretion of 22 nm HBsAg particle revealed a great potential for this organism and its growth in the presence of glucanase to secrete other important mammalian proteins which have been difficult to secrete in other yeast systems, such as virus coat proteins, tissue plasminogen activator, bovine chymosin and so on.

The $\beta$-1,3-glucanase for permeabilizing the yeast cell wall can be produced in large quantity by recombinant DNA technology. Accordingly, the production of HBsAg in secreted form from H. polymorpha and other yeasts is practical. A more sophisticated and economical way will be to clone the $\beta$-1,3-glucanase into the genome of H. polymorpha and other yeasts so that the recombinant yeast will be permanently able to secrete the HBsAg particles and other high molecular weight proteins or particles.

## IN THE DRAWINGS

Figure 1 shows the construction of the expression vectors pMHS and pMHS2.
Figure 2 shows the accumulation of HBsAg in the culture medium.
Figure 3 shows the electron micrograph (115,500X) of HBsAg particles produced from the medium.
Figure 4 shows the immunoblot of HBsAg proteins excreted into the medium.

4

## DETAILED DESCRIPTION OF THE INVENTION

The present invention relates to a process through which yeast cells may be permeabilized to allow the excretion of secreted material into the growth medium by adding enzymes directly to the culture medium. Preferably, the enzyme to be used is a glycanase, more preferably $\beta$-1,3-glucanase, although it is to be noted that the use of enzymes such as zymolyase, NOVOzyme™, $\beta$-glucuronidase and lyticase may also be contemplated. The enzyme may also be provided intracellularly by being genetically synthesized within the host yeast.

In order to illustrate the process of the present invention, there is described the characterization of HBsAg expressed and secreted from H. polymorpha. Evidence and methodology for the excretion of the assembled HBsAg particles into the culture medium are also presented.

According to the present invention, the yeast culture to be employed should be suitable for growth on carbon-containing substrates under aqueous fermentation conditions. Examples of suitable species include: Hansenula minuta, Hansenula saturnus, Hansenula californica, Hansenula silvicola, Hansenula polymorpha, Hansenula capsulato, Hansenula glucozyma, Hansenula nonfermentans, Hansenula philodenro, Saccharomyces cerevisiae, Saccharomyces fragilis, Saccharomyces rosei, Saccharomyces elegans, Saccharomyces lactis, Schizosaccharomyces pombe and Pichia patoris. If desired, mixtures of two or more species of yeasts can be employed.

Presently preferred for the protein production are those Hansenula polymorpha and Saccharomyces cerevisiae which do grow suitably on oxygenated-hydrocarbon feed stocks.

Hansenula polymorpha is a methylotrophic yeast capable of using methanol as a sole carbon source. The first enzyme required for growth on the methanol is methanol oxidase (MOX) which, together with other enzymes involved in methanol metabolism, is induced by methanol and repressed by glucose. Under appropriate induction conditions, this enzyme, located in the peroxisome, accounts for more than 15% of total cellular protein (Gleeson, M.A., and P.E. Sudbery, The Methylotrophic Yeasts, Yeast 4:1-15, (1988)). This property makes its promoter attractive for expression of heterologous genes. The MOX gene has been cloned and sequences (Ledeboer, A.M., L. Edens, J. Maat, C. Visser, J.W. Bos, C.T. Verrips, Z. Janowics, M. Eckart, R. Roggenkamp, and C.P. Hollenberg, Molecular cloning and characterization of a gene coding for methanol oxidase in Hansenula polymorpha, Nucl. Acids Res. 13:178-180, (1985)). To develop the Hansenula polymorpha expression system, the promoter of the methanol oxidase (MOX) gene was isolated and used to direct the expression of HBsAg proteins.

## a) Construction of the expression vectors

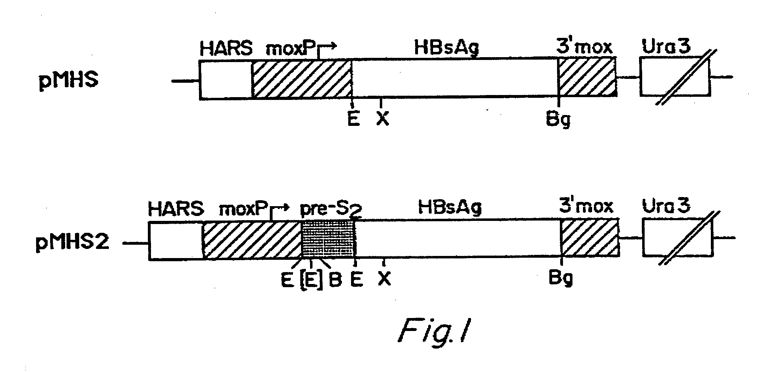

Two expression vectors have been used here for the demonstration of the present invention. As illustrated in figure 1, one of the vectors, termed pMHS, expresses the 226-residue S protein, and the other, pMHS2, encodes the same S polypeptide plus an additional 55 aminoterminal residues derived from the so-called preS region. Both the S and preS2 genes were fused at their 5' end to the promoter of the MOX gene and at their 3' end to the transcription terminator of the MOX gene. The vectors also contained the H. polymorpha autonomous replicon (HARS) (Roggenkamp, R., H. Hansen, M. Eckart, Z. Janowicz and C.P. Hollenbert, Transformation of the methylotrophic yeast Hansenula polymorpha by autonomous replication and integration vectors, Mol. Gen. Genet. 202:302-308 (1986)), the Ura-3 gene of S. cerevisiae for complementary selection, and a sequence derived from pUC19 plasmid for transformation and replication in E. coli.

To construct these two expression vectors, a HindIII-SacI fragment of the MOX gene was cloned into a pUC19 derivative in which the EcoRI site had been destroyed. An EcoRI site was inserted immediately upstream of the initiation codon of the MOX gene by site-directed mutagenesis. The HBsAg gene (Moriarty, A.M., B.H. Hoyer, J.W. Shih, J.L. Gerin, and D.H. Hamer, Expression of the hepatitis B virus surface antigen gene in cell culture by using a simian virus 40 vector, Proc. Natl. Acad. Sci. USA, 78: 2606-2610, (1981)) sequences located between an EcoRI site at position 1 and an HpaI site at position 961, which has been converted to BglII by a linker insertion, was cloned between the EcoRI site (codon 6) and BglII (codon 655) sites of the MOX gene, resulting in deletion of nearly the entire coding sequence of the MOX gene. The resultant plasmid was termed pMXHB. For construction of the pMHS plasmid, the extra preS sequence in the pMXHB was looped out by site-directed mutagenesis. To construct the pMHS2 plasmid, the additional sequence in the preS2 region from the first codon (ATG) to the fourth codon (AAT) at the EcoRI site was inserted into the pMXHB by synthetic oligonucleotides. In both vectors, a BglII fragment (about 1 Kb) of the Ura-3 gene (J.D. Boeke, F. LaCroute and G.R. Fink, A positive selection for mutants lacking orotidine-5´-phosphate decarboxylase activity in yeast: 5-fluoro-orotic acid resistance, Mol. Gen. Genet. 197, 345-346, (1984)) was inserted into the SacI site by blunt-end ligation. The HARS sequence, a 470 bp of a SalI-HindIII fragment was finally engineered into the expression vector. In Figure 1, E, EcoRI; B, BamHI; X, XbaI; Bg, Bg1II.

b) Detection of HBsAg in the periplasm following β-1,3-glucanase digestion

The expression vectors, pMHS and pMHS2, were transformed into a uracil auxotroph of H. polymorpha, Ura3-1, which can be rescued by the Ura-3 gene of S. cerevisiae. Transformation was performed either by the proroplasting method or by the LiCl procedure, both of which were originally developed for S. cerevisiae (Das, S., E. Kellerman, and C.P. Hollenbert, Transformation of Kluyveromyces fragilis, J. Bacteriol. 158:1165-1167 (1984); Hinnen, A., J.B. Hicks, and G.R. Fink, Transformation of yeast, Proc. Natl. Acad. Sci. USA 75, 1929-1933 (1978)).

The transformants were initially grown in a synthetic medium (Gleeson, M.A., and P.E. Sudbery, The methylotrophic Yeasts, Yeast 4:1-15 (1988)) lacking uracil overnight at 37°C with shaking. To synthesize HBsAg, cells were harvested by centrifugation and resuspended into a fresh YEPD medium in which the 2% glucose was replaced by 0.5% methanol as a sole carbon source. Upon depletion of the intracellular glucose pool, the methanol regulated MOX promoter is induced to express HBsAg. To determine the level of HBsAg synthesis and its localization, the culture medium and the cytoplasm, which was prepared by vortexing collected cells with glass beads, were assayed. The amount of HBsAg was measured using an enzyme-linked immunoassay which detects HBsAg 22 nm particles (E.I.A. AUZYME II, Abbott Laboratories). After three days of induction, approximately 500 ng/ml of HBsAg were detected in the cytoplasm of transformants from both plasmids. As expected, no appreciable levels of HBsAg proteins were found in the culture media (less than 20 ng/ml).

As the present invention demonstrates, HBsAg particles trapped by the yeast cell wall, could be secreted into the medium following permeabilization of the cell wall by treatment with β-1,3-glucanase. β-glucanase, one of glucanases, was used to digest glucan which forms part of the structural network of the yeast cell wall. β-1,3-glucanase was purified as described by Scott and Schekman (Lyticase: Endoglucanase and protease activities that act together in yeast cell lysis, J. Bacteriology, 142:414-423, 1980). To examine the effect of glucanase on the secretion of HBsAg, cells which had been under induction conditions for three days were collected and fractionated into a periplasmic fraction and a cytoplasmic fraction as follows. Cells from identical 10 ml cultures were pelleted and resuspended into buffer A (1.2 M sorbitol, 50 mM phosphate, pH 7.5 and 1 mM phenylmethylsulfonylfluorid [PMSF]) with and without β-1,3-glucanase (10 µg/ml); samples and controls were incubated at 37°C for 1 hr then centrifuged. The supernatants from this step constituted the periplasmic fraction. The cytoplasmic fraction was obtained by vortexing the pellets from the periplasmic step in buffer A with glass beads followed by removal of cell debris by centrifugation. Both fractions were assayed for the level of HBsAg and, as a control for the fractionation method, the activity of a cytoplasmic enzyme, formate dehydrogenase (FDH), was determined as described by L.P. Van Dijken, R. Otto and W. Harder (Arch. Microbiol. 111:137-144, (1976)). As shown in Table I, while less than 1% of the total cytoplasmic marker (FDH) activity was found in the periplasmic fractions, the same fractions contained 25% and 54% of total HBsAg protein produced by pMHS and pMHS2, respectively. These results shown that β-1,3-glucanase has caused the release of considerable amounts of HBsAg into the solution without causing much leakage of cytoplasmic components.

TABLE 1

| Distribution of HBsAg and formate dehydrogenase (FDH) in H. polymorpha cultures with and without glucanase treatment. | | | |
|---|---|---|---|
| | Cytoplasm | Periplasm | % in Periplasm |
| pMHS No Glucanase | | | |
| HBsAg<br>FDH | 516<br>na | 18<br>na | 3<br>- |
| pMHS Plus Glucanase | | | |
| HBsAg<br>FDH | 378<br>3,225 | 126<br>12 | 25<br><1 |
| pMHS2 No Glucanase | | | |
| HBsAg<br>FDH | 514<br>na | 22<br>na | 4<br>- |
| pMHS2 Plus Glucanase | | | |
| HBsAg<br>FDH | 264<br>3,326 | 308<br>10 | 54<br><1 |
| HBsAg is in ng/ml, FDH activity is 340 nm/min/ml.<br>"na", not assayed. | | | |

Electron microscopic examination of the glucanase treated cells confirmed that the cell wall had not been removed. Treated cells were also found to be resistant to lysis by hypotonic solutions or 0.1% Triton X-100, as were untreated cells. In addition, the viability of treated cells was about the same as that of mock treated controls, as judged by their plating efficiency. These data prove that glucanase treatment, while not adversely affecting the physiology of yeast, allows HBsAg proteins in the periplasm to be released into the medium.

c) Secretion and production of HBsAg in the culture medium.

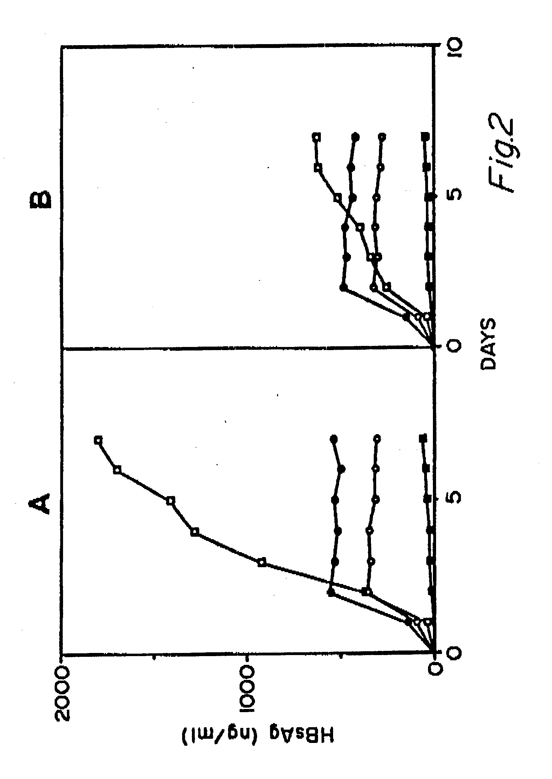

For utilization of the present invention for the secretion of HBsAg particles, $\beta$-1,3-glucanase (10 ug/ml) plus PMSF (1 mM) were added directly to the culture medium at the beginning of induction (5 x $10^8$ cells/ml) (Figure 2, open symbols). As a control, glucanase was omitted from parallel cultures (Figure 2, closed symbols). Cultures were sampled daily (1 ml) and the amount of HBsAg in the cytoplasm and in the medium was determined as before. Under these conditions, the cell number, for both treated and untreated cultures, doubled after the first day of induction and remained essentially unchanged at approximately $10^9$ cells/ml for the remainder of the experiment; an indication that glucanase treatment does not interfere with cells growth. Figure 2 shows the amount of S (Fig. 2B) and preS2 (Fig. 2A) protein in the medium (squares in Fig. 2) and in the cytoplasm (circles in Fig. 2) for treated and untreated cultures as a function of time. For both proteins, the absence of glucanase resulted in less than 40 ng/ml of antigen detected in the medium, while the intracellular concentration increased to about 500 ng/ml without two days and remained at about the same level for the last 5 days (Fig. 2). In the presence of glucanase, however, the amount of both types of antigen in the medium began to increase steadily following a lag of one day. While the extracellular concentration of HBsAg was increasing, the intracellular level of antigen remained constant at about 350 ng/ml, a level comparable to that of the untreated cultures.

More interestingly, after two days of induction, the total output of the system (the sum of intra- and extra-cellular concentrations) in the presence of glucanase is significantly higher than that in its absence. For example, at day 6, the respective values are 2,008 ng/ml versus 552 ng/ml for the preS2 and 910 ng/ml versus 490 ng/ml for the S. Consequently, the amount of preS2 in the medium (1699 ng/ml) was over 3 times the initial intracellular level (500 ng/ml) due to its continuous synthesis and secretion. These results show that using glucanase to permeabilize the yeast cell wall not only allows the secretion of HBsAg into the medium, but also substantially increases the overall yield of antigen production. It appears that accumulation of the HBsAg protein inside the cell or in the periplasm inhibits further synthesis of the antigen, and removal of the HBsAg by secretion into the medium results in greater overall output of antigen.

The rate of secretion, calculated from an average slope for the preS2 (Fig. 2A) and the S (Fig. 2B) curve, is 320 ng/$10^9$ cells/day and 100 ng/$10^9$ cells/day, respectively. The elevated levels for preS2 over S may be a consequence of a more efficient transfer process to the outside of the cell membrane prior to release into the medium.

d) Characterization of the secreted HBsAg.

In order to characterize the molecular form of the antigens secreted into the medium, the transformants were induced in the presence of β-glucanase for two days. Media samples from cultures secreting preS or S protein were subjected to two types of molecular size analyses: ultrafiltration using a membrane with a molecular weight exclusion limit of 100,000 or ultracentrifugation through a 7% sucrose cushion in manner which causes pelleting of the 22 nm HBsAg particles but not the monomers. Less than 5% of the input amount of HBsAg was detected in the filtrate, or in the centrifugation supernatant, indicating that the HBsAg proteins exported into the medium were present as large aggregates.

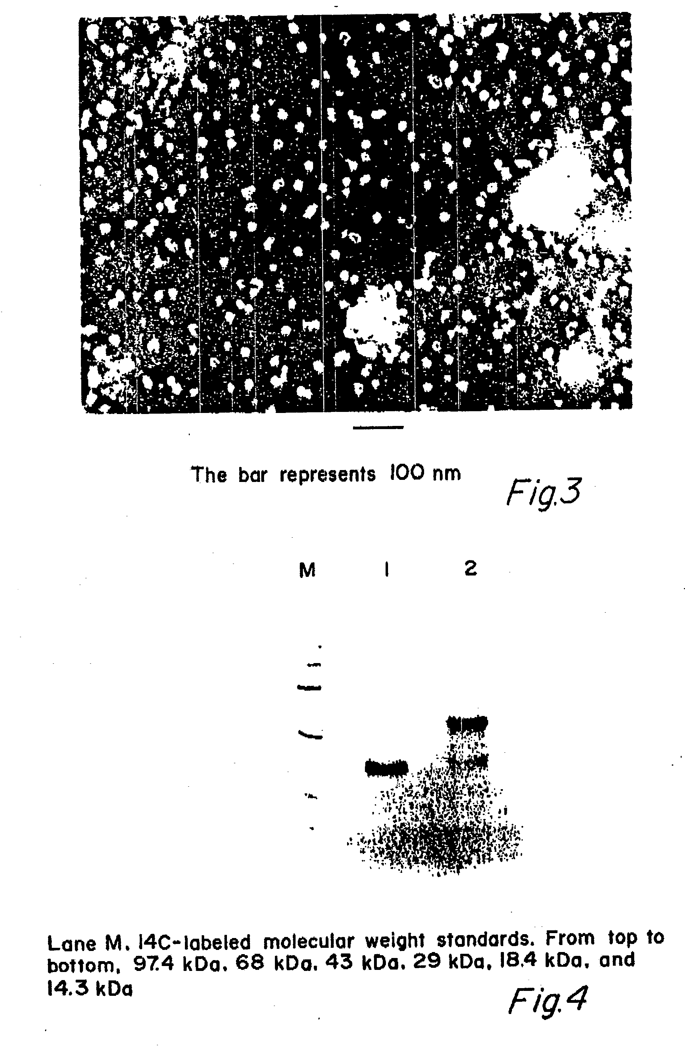

Since assembled HBsAg particles in other yeasts have only been obtained from broken cells, a possibility remains that the 22 nm particles are formed during the process of isolation (D.E. Wampler, E.D. Lehman, J. Boger, W.J. McAleer, and E.M. Scolnick, Proc. Natl. Acad. Sci. USA 82:6830-6834, (1985)). To determine whether the HBsAg proteins secreted into the medium from H. polymorpha is in the form of particles with ordered structure or is merely large aggregates, the culture medium (200 ml) of induced transformants in the presence of glucanase was concentrated to 10 ml by ultrafiltration using YM 100 (Amicon) and the concentrated HBsAg were partially purified by Aerosil 380 gel (Degussa Corp.) as described by Pillot et al. (J. Clin. Microbiol. 4:205-207, (1976)). The HBsAg samples were negatively stained with uranylacetate and examined by electron microscopy. The micrograph shown in Figure 3 reveals that the excreted material is in the form of discrete particles about 22 nm of diameter similar to those found in serum of human patients.

The HBsAg proteins secreted into the medium were further characterized by immunoblotting. About 200 ng of HBsAg secreted into the medium of transformants from pMHS or pMHS2 were reduced, denatured and separated through a 14% SDS-polyacrylamide gel. As the autoradiograph in Figure 4 shows, pMHS transformants secrete a single protein with an apparent molecular mass of about 23 kDa (Fig. 4, lane 1), a value in agreement with the molecular weight without glycosylation observed in other recombinant yeasts expressing the S form of HBsAg. In the culture medium of pMHS2 transformants, a prominent immuno-reactive protein component at about 32 kDa was found (Fig. 4, lane 2). The molecular weight without glycosylation for preS2 is expected to be less than 31 kDa. The observed 32 kDa molecular mass is probably a glycosylated form of the preS2 protein as previously observed in recombinant animal cells, as well as in yeast. The minor component migrating just behind the 23 kDa point is presumably a degradation product. These data show that the S and preS2 proteins secreted into the culture medium and identified by their immunoreactivity are not degraded components.

Thus, the present invention provides a novel procedure involving an enzyme capable of permeabilizing the yeast cell wall and thereby allowing excretion of produced biological products trapped in the periplasm into the culture medium. β-1,3-glucanase, present in the culture medium of H. polymorpha not only effects the secretion of the 22 nm particles of HBsAg into the medium, but also significantly increases the yield of antigen production. The ability to secrete complex macromolecules into the culture medium makes yeast cells an efficient secretion system comparable to animal cells, but more economical and less potentially hazardous for the production of recombinant products, particularly, those for use in humans.

**Claims**

1. A process for the excretion and production of biological products by yeast cells, said process comprising:
- continuously growing said yeast cells in a growth medium in the presence of at least one enzyme capable of permeabilizing the cell wall of said yeast cells, thereby allowing release of said biological products from the inside of said yeast cell wall into said growth medium and
- recovering the desired products from said growth medium.

2. A process according to claim 1, wherein the enzyme capable of permeabilizing the yeast cell wall is a glucanase.

3. A process according to claim 2, wherein the glucanase is $\beta$-1,3-glucanase.

4. A process according to claim 1, wherein the enzyme is selected from the group consisting of zymolyase, NOVOzyme™, $\beta$-glucuronidase and lyticase.

5. A process according to claim 1, wherein the enzyme capable of permeabilizing the yeast cell wall is extraneously added into the growth medium.

6. A process according to claim 1, wherein the enzyme capable of permeabilizing the yeast cell wall is intracellularly provided in a genetic form synthesized within the host yeast.

7. A process according to claim 1, wherein the yeast is selected from the group consisting of Hansenula polymorpha, Pichia pastoris, Saccharomyces or Schizosaccharomyces pombe.

8. A process according to claim 1, wherein said biological product is a hepatitis B surface antigen.

9. A process according to claim 1, wherein said biological products are selected from the group consisting of viral coat proteins, tissue plasminogen activator and bovine chymosin.

*Fig.1*

EP 0 389 377 A1

Fig.2

EP 0 389 377 A1

The bar represents 100 nm

*Fig.3*

M       1       2

Lane M. 14C-labeled molecular weight standards. From top to bottom, 97.4 kDa, 68 kDa, 43 kDa, 29 kDa, 18.4 kDa, and 14.3 kDa

*Fig.4*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 061 250 (BIOGEN N.V.) <br> * Page 5, lines 15-30; page 6, line 19 - page 7, line 16; example 1 * <br> --- | 1,5,7-9 | C 12 P 21/02 <br> C 12 N 1/38 <br> C 12 N 1/06 |
| X | EP-A-0 155 189 (GENENTECH INC.) <br> * Abstract * <br> ----- | 1,7-9 | |

TECHNICAL FIELDS
SEARCHED (Int. Cl.5)

C 12 P
C 12 N

The present search report has been drawn up for all claims

| Place of search | Date of completion of the search | Examiner |
|---|---|---|
| THE HAGUE | 13-06-1990 | VAN PUTTEN A.J. |

EPO FORM 1503 03.82 (P0401)